# EUROPÄISCHE PATENTSCHRIFT

(11) **EP 2 710 025 B1**
(45) Veröffentlichungstag und Bekanntmachung des Hinweises auf die Patenterteilung: **20.05.2015**
(21) Anmeldenummer: 12711564.0
(22) Anmeldetag: 21.03.2012
(51) Int. Cl.: C07K 1/16, A61K 47/48

(54) **VERFAHREN ZUR AUFTRENNUNG EINES GEMISCHES AUS EINEM PROTEIN UND SEINEM REAKTIONSPRODUKT MIT EINEM POLYALKYLENGLYKOL**
METHOD FOR SEPARATING A MIXTURE OF A PROTEIN AND THE REACTION PRODUCT THEREOF WITH A POLYALKYLENE GLYCOL
PROCÉDÉ POUR SÉPARER UN MÉLANGE CONSTITUÉ D'UNE PROTÉINE ET DE SON PRODUIT DE RÉACTION À L'AIDE D'UN POLYALKYLÈNE GLYCOL

(30) Priorität: 19.05.2011 DE 102011101995
(43) Veröffentlichungstag der Anmeldung: 26.03.2014
(73) Patentinhaber: Sartorius Stedim Biotech GmbH, 37079 Göttingen (DE)
(72) Erfinder: DEMMER, Wolfgang, 37077 Göttingen (DE); VILLAIN, Louis, 30451 Hannover (DE); HÖRL, Hans-Heinrich, 37120 Bovenden (DE)
(74) Vertreter: Stehl, Astrid
(86) Internationale Anmeldenummer: PCT/EP2012/001230
(87) Internationale Veröffentlichungsnummer: WO 2012/156000

(56) Entgegenhaltungen:
- WO-A1-2005/029065
- WO-A2-2008/057683
- DE-A1-102008 018 732
- US-A1- 2005 089 952
- FEE C J ET AL: "PEG-proteins: Reaction engineering and separation issues", CHEMICAL ENGINEERING SCIENCE, OXFORD, GB, Bd. 61, Nr. 3, 1. Februar 2006 (2006-02-01), Seiten 924-939, XP025012432, ISSN: 0009-2509, DOI: 10.1016/J.CES.2005.04.040 [gefunden am 2006-02-01] in der Anmeldung erwähnt

## Beschreibung

Die vorliegende Erfindung betrifft ein Verfahren zur Auftrennung eines Gemisches aus einem Protein und seinem Reaktionsprodukt mit einem Polyalkylenglykol.

Bei der Verwendung therapeutischer Proteine in der Medizin werden die Proteine häufig mit einem Polyethylenglykol funktionalisiert, um die biologische Halbwertszeit und somit die Wirkdauer nach der Aufnahme in den Blutkreislauf des Patienten zu verlängern. Als Synonym für ein Protein, welches mit einer Polyethylenglykol-Einheit funktionalisiert ist, wird auch häufig der Begriff eines einfach, zweifach oder mehrfach "pegylierten" Proteins verwendet, wobei das Proteinmolekül wahlweise mit einer, zwei oder mehreren Polyethylenglykol-Polymerketten funktionalisiert ist. Pegylierte Proteine werden aufgrund ihres größeren hydrodynamischen Radius langsamer über die Nieren elimiert als die entsprechenden nicht pegylierten Proteine. Weiterhin wird für den Patienten durch die Pegylierung eine unerwünschte Nebenwirkung des Proteins als Antigen reduziert.

Effizienten Verfahren zur schnellen Bestimmung des Anteil eines einfach, zweifach und/oder mehrfach pegylierten Proteins in einem Stoffgemisch kommt in der medizinischen und biopharmazeutischen Schnellanalytik zunehmende Bedeutung zu, weil mit diesen Verfahren in kurzer Zeit zuverlässige Aussagen über die quantitative Zusammensetzung derartiger Gemische und über den Pegylierungsgrad der darin enthaltenen Proteine erhalten werden.

In den letzten Jahren haben sich verschiedene Verfahren etabliert, mit welchen Proteine über ihre Aminogruppen mit chemisch aktivierten, beispielsweise Aldehydgruppen aufweisenden Polyalkylenglykolen zu Schiffschen Basen (Iminen) umgesetzt werden können, wobei die Imin-Gruppen des Proteins nachfolgend zu pegylierten Aminogruppen reduziert werden. Je nachdem, mit wie vielen Aminogruppen eines Proteins diese Reaktionssequenz mit einem Polyethylenglykol abläuft, resultiert ein einfach, zweifach oder mehrfach "pegyliertes" Protein.

WO 2007/056191 A2 offenbart ein Verfahren zur Reinigung von Nukleotiden, welche eine Glycineinheit aufweisen, deren Aminogruppe mit einer Polyethylenglykol-Einheit funktionalisiert ist. Das einfach pegylierte Nukleotid kann durch eine Kombination aus Membranfiltration (Reverse-Osmose- oder Nanofiltration), "Size-Exclusion"-Chromatographie mit Polyacrylamidharzen und Ionenaustauschchromatographie mit Q-Sephacose^{®} von dem nicht abreagierten Pegylierungsreagenz abgetrennt werden. Dieses Dokument offenbart kein Verfahren zur Auftrennung von Gemischen, die ein, zwei- und mehrfach pegylierte Nukleotide bzw. Proteine enthalten, in die Einzelkomponenten.

WO 2008/154639 A2 offenbart ein Verfahren zur Reinigung von einfach pegylierten Nukleotiden in hoher Ausbeute und Reinheit, wobei das einfach pegylierte Nukleotid durch Anionenaustausch-Chromatographie unter Verwendung von Q-Sepharose^{®}-Gelen, Mustang^{®}-Q- oder Sartobind^{®}-Q-Membranadsorbern und nachfolgende Ultrafiltration bzw. Tangentialfluß-Filtration von dem nicht pegylierten Nukleotid und von dem nicht abreagierten Pegylierungsreagenz abgetrennt wird. Auch dieses Dokument offenbart kein Verfahren zur Auftrennung von Gemischen, die ein, zwei- und mehrfach pegylierte Nukleotide bzw. Proteine enthalten, in die Einzelkomponenten.

WO 2006/011839 A1 offenbart ein Verfahren zur Reinigung eines einfach bzw. mehrfach pegylierten, nicht näher spezifizierten 30-kDa-Proteins unter Verwendung eines Chromatographiegels auf Basis eines vernetzten Copolymers aus Allyldextran und N,N-Methylenbisacrylamid, wie z. B. Sephacryl^{®} S 500. Auf der Oberfläche des Chromatographiegels sind ionenaustauschende oder hydrophobe Gruppen bzw. Affinitätsgruppen oder metallchelatbildende Gruppen immobilisiert. Mit Hilfe des Verfahrens ist es möglich, das einfach pegylierte Protein von dem mehrfach pegylierten Protein abzutrennen.

WO 2005/029065 A1 offenbart Chromatographiematrizen, welche aus vernetzter Agarose bestehen, auf dessen Oberfläche Polymerketten auf Basis von Polyacrylsäure fixiert sind. Mit diesen Chromatographiematrizen werden einfach pegylierte Proteine von den Edukten einer Pegylierungsreaktion, d. h. von dem nicht umgesetzten Pegylierungsreagenz und von den nicht pegylierten Proteinen, abgetrennt. Essentiell für das Gelingen des Verfahrens sind die protonenliefernden Carbonsäurefunktionen der Polyacrylsäure-Polymerketten. Für die in WO 2005/029065 A1 offenbarten Chromotographiematrizen wurde festgestellt, daß bei einer Gradienten-Elution die Bindungswechselwirkung zwischen der Matrix und den Komponenten des Reaktionsgemisches in der Reihenfolge Pegylierungsreagenz > einfach pegylierte Komponente > nicht pegylierte Komponente abnimmt. Ebenso wie die vorgenannten Dokumente offenbart auch dieses Dokument kein Verfahren zur Auftrennung von Gemischen, die ein- und mehrfach pegylierte Proteine enthalten, in die Einzelkomponenten.

US 2005/0089952 A1 offenbart die Abtrennung eines nicht pegylierten Proteins, z. B. Lysozym, von seinen einfach und mehrfach pegylierten Reaktionsprodukten unter Verwendung von Membranen aus Polyethersulfon bzw. regenerierter Cellulose in einem Tangentialfluß- oder Diafiltrationsverfahren. Entscheidend für die Abtrennung des nicht pegylierten Proteins von den pegylierten Komponenten ist ein "Molecular-Weight-Cut-Off"-Wert (MWCO-Wert) der Membranen von mindestens 30 kDa. Unter dieser Vorraussetzung passiert das nicht pegylierte Lysozym im Permeatstrom die Membran, während die pegylierten Lysozym-Spezies zu 97-99,2 % von der Membran zurückgehalten werden. Ziel des aus US 2005/0089952 A1 bekannten Trennverfahrens ist die effektive und möglichst quantitative Abtrennung des nicht pegylierten Proteins von den einfach bzw. mehrfach pegylierten Protein-Spezies, damit das nicht pegylierte Protein zurückgewonnen und dem Reaktionsbehälter für die Pegylierung wieder zugeführt werden kann. Dementsprechend enthält auch dieses Dokument keine Offenbarung, wie mit den vorgenannten Membranen ein Gemisch aus ein- und mehrfach pegylierten Proteinen in diese Spezies aufgetrennt werden kann.

J. R. Molek und L. Zydney offenbaren in Biotechnol. Prog. 2007 (23), 1417-1424, die Abtrennung von pegyliertem Lactalbumin von nicht pegyliertem Lactalbumin und von weiteren Nebenprodukten mittels neutraler oder mit Sulfonsäuregruppen funktionalisierter Ultrafiltrationsmembranen auf Basis von regenerierter Cellulose und mit einem MWCO-Wert von 30 bzw. 100 kDa.

C. J. Fee et al. offenbaren in ihrem Übersichtsartikel aus Chemical Engineering Science, 61, 2006, 924-939, verschiedene Trennverfahren, darunter Größenausschluß-, Affinitäts-, Hydrophobe-Interaktions-, Kationenaustausch-, Metallchelat-, "Reversed Phase"- und Anionenaustauschchromatographieverfahren, um pegylierte, therapeutische Proteine von ihren nicht pegylierten Protein-Edukten abzutrennen.

Der vorliegenden Erfindung liegt die Aufgabe zugrunde, ein Verfahren zur Auftrennung von Gemischen aus einem Protein und seinen einfach oder mehrfach durch Polyalkylenglykol funktionalisierten Reaktionsprodukten bereitzustellen, welches es erlaubt, auf effiziente und kostengünstige Weise sowohl das Protein als auch die durch das Polyalkylenglykol funktionierten Proteine einzeln abzutrennen und welches gleichzeitig erlaubt, schnell und kostengünstig festzustellen, in welchen quantitativen Verhältnissen das Protein und seine Reaktionsprodukte in dem Gemisch vorliegen.

Diese Aufgabe wird durch das Verfahren nach Anspruch 1 und seine in den abhängigen Ansprüchen gekennzeichneten Ausführungsformen gelöst. Insbesondere wird erfindungsgemäß ein Verfahren zur Auftrennung der vorgenannten Gemische aus Proteinen bereitgestellt, das die vorgenannten Anforderungen erfüllt. Dabei wurde überraschend gefunden, daß eine Auftrennung von Gemischen aus einem Protein und seinen durch Polyalkylenglykol funktionalisierten Reaktionsprodukten in die Einzelkomponenten durch die Verwendung einer mikroporösen Celluloseacetatmembran möglich ist.

Dementsprechend betrifft die Erfindung ein Verfahren zur Auftrennung eines Gemisches aus einem Protein und seinem Reaktionsprodukt mit einem Polyalkylenglykol, umfassend die Schritte:
a) Bereitstellen eines Gemisches umfassend das Protein und das Reaktionsprodukt in einem Fluid,
b) Kontaktieren des Gemisches aus Schritt a) mit einer mikroporösen Cellulose-acetatmembran unter Adsorbieren des Reaktionsprodukts an der Celluloseacetatmembran, wobei das Protein nicht an der Celluloseacetatmembran adsorbiert wird,
c) Abtrennen des Proteins von der Celluloseacetatmembran und
d) Desorbieren des Reaktionsprodukts von der Celluloseacetatmembran,
wobei das Reaktionsprodukt aus der Gruppe, die das einfach oder mehrfach durch das Polyalkylenglykol funktionalisierte Protein oder Gemische davon umfaßt, ausgewählt ist.

Der Begriff "Polyalkylenglykol" umfaßt erfindungsgemäß Polyether der allgemeinen Formel HO-[R-O-]ₙH, wobei die Alkylengruppe R ein zweiwertiger Rest ist, welcher an zwei verschiedenen C-Atomen zwei Wasserstoffatome weniger aufweist als das zugrundeliegende Alkan H-R-H, und wobei n eine natürliche Zahl größer gleich zwei ist. Die Alkylengruppe R kann als Substituenten Wasserstoffatome, Alkylreste und/oder Arylreste aufweisen, wobei die Alkyl- bzw. Arylreste ihrerseits weitere beliebige Substituenten, z. B. funktionelle Gruppen mit Heteroatomen, aufweisen können. Das Polyalkylenglykol kann erfindungsgemäß ein linearer oder verzweigter Polyether sein.

"Protein" im Sinne der vorliegenden Erfindung umfaßt jedes Oligo- bzw. Polypeptid und übergeordnete Primär-, Sekundär- bzw. Tertiärstrükturen eines Polypeptids, welche mit einem Polyalkylenglykol umgesetzt werden können. Unter "Protein" werden erfindungsgemäß auch Aggregate aus mindestens zwei der vorgenannten Proteine verstanden.

Der Begriff "Reaktionsprodukt" eines Proteins mit einem Polyalkylenglykol umfaßt das unmittelbare oder mittelbare Umsetzungsprodukt einer funktionellen, reaktiven Gruppe des Proteins mit einer komplementären, funktionellen Gruppe des Polyalkylenglykols. Insbesondere wird erfindungsgemäß unter dem vorgenannten Reaktionsprodukt ein Produkt verstanden, welches aus der Umsetzung von Aminogruppen des Proteins mit in das Polyalkylenglykol durch Oxidation von -CH-OH-Gruppen einführbaren Aldehydgruppen resultiert. Bei dieser Umsetzung werden zunächst Imine ("Schiffsche Basen") generiert, die in einem Folgeschritt, z. B. durch Reduktion, zu durch einen Polyether-Substituenten funktionalisierten Amingruppen des Proteins umgesetzt werden können.

Je nach Anzahl der in dem Protein vorhandenen Amingruppen, die mit komplementären, funktionellen Gruppen des Polyalkylenglykols reaktionsfähig sind, resultiert als Reaktionsprodukt ein Protein, welches nur eine durch einen Polyether-Substituenten funktionalisierte Amingruppe hat, wobei dieses Protein erfindungsgemäß ein "einfach durch das Polyalkylenglykol funktionalisiertes Protein" ist, oder es resultiert ein Protein, welches zwei oder mehr durch einen Polyether-Substituenten funktionalisierte Amingruppen hat, wobei dieses Protein ein "mehrfach durch das Polyalkylenglykol funktionalisiertes Protein" ist.

Erfindungsgemäß wird unter einem "Gemisch aus einem Protein und seinem Reaktionsprodukt mit einem Polyalkylenglykol" ein Gemisch aus dem Protein und mindestens einem seiner vorgenannten, einfach oder mehrfach durch einen Polylalkylenether-Substituenten funktionalisierten Umsetzungsprodukte verstanden.

Bevorzugt wird für die Durchführung des erfindungsgemäßen Verfahrens als Polyalkylenglykol ein Polyethylenglykol mit einem mittleren Molekulargewicht von 5 bis 30 kDa eingesetzt.

Polyethylenglykole mit aktivierten Aldehydgruppen, die mit freien Amingruppen des Proteins zu Iminen umgesetzt werden können, welche nachfolgend zu ein- oder mehrfach durch einen Polyethylenether-Substituenten funktionalisierten Amingruppen reduziert werden können, sind erfindungsgemäß besonders bevorzugt. Diese durch eine sogenannte "Pegylierung" funktionalisierten Proteine werden nachfolgend synonym auch als mono-, di-, tri- bzw. multipegylierte Proteine bezeichnet. Verfahren zur Pegylierung der Aminogruppen von Proteinen sind von M. R. Sherman et al. in "Conjugation of High-molecular Weight Poly(ethylene glycol) to Cytokines: Granulocyte-Macrophage Colony-stimulating Factors as Model Substrates" in der Monographie "Poly (ethylene glycol) Chemistry and Biological Applications", ACS Symposium Series 680, Herausgeber: J. M. Harris, S. Zalipsky, American Chemical Society 1997, S.155-169, ISBN 0-8412-3537-6, beschrieben worden.

Als "Fluid" kommt erfindungsgemäß jede mit dem Protein und seinem Reaktiönsprodukt kompatible Flüssigkeit in Betracht, in welcher das Protein und sein Reaktionsprodukt als Gemisch bereitgestellt werden können, d. h. insbesondere wäßrige Lösungen von (an)organischen Salzen, denen wahlweise mindestens ein Alkanol zugemischt werden kann.

Der Begriff "mikroporöse Celluloseacetatmembran" bezeichnet im Rahmen der vorliegenden Erfindung Celluloseacetatmembranen mit einer Porengröße von 0,1 bis 15 µm, vorzugsweise 0,1 bis 5 µm und mehr bevorzugt 0,2 bis 0,45 µm. Die Bestimmung der Porengröße kann mit einem sogenannten "Capillary Flow Porometry Test" (Capillary Flow Porometer 6.0, CAPWIN Software System, Fa. Porous Materials Inc.) durchgeführt werden. Die mikroporöse Celluloseacetatmembran kann in jeder Form vorliegen, die geeignet ist, daß die inneren und äußeren Oberflächen der Membran mit dem Gemisch aus dem Protein und seinem Reaktionsprodukt mit einem Polyalkylenglykol kontaktiert werden. Beispielsweise kann die mikroporöse Celluloseacetatmembran in ein Membranadsorbermodul integriert sein. Geeignete Membranadsorbermodule sind beispielsweise aus DE 102 36 664 A1 bekannt.

Bei einer weiteren bevorzugten Ausführungsform der Erfindung ist die Celluloseacetatmembran durch ein Vlies oder Gewebe verstärkt, um die mechanische Stabilität der Membran zu erhöhen.

Gemäß einer weiteren bevorzugten Ausführungsform der Erfindung besteht die Cellulose-acetatmembran aus Cellulosemonoacetat, Cellulosediacetat, Cellulosetriacetat oder Gemischen davon.

Gemäß einer bevorzugten Ausführungsform des erfindungsgemäßen Verfahren ist das Reaktionsprodukt des Proteins mit dem Polyalkylenglykol ein Gemisch, welches das einfach und mehrfach durch das Polyalkylenglykol funktionalisierte Protein umfaßt, und wobei in Schritt d) die durch das Polyalkylenglykol funktionalisierten Proteine nacheinander einzeln mit einem Elutionsmittel von der Celluloseacetatmembran desorbiert werden.

Bevorzugt wird als Elutionsmittel in Schritt d) eine wäßrige Lösung eines anorganischen Ammonium-, Alkali- bzw. Erdalkalimetallsalzes und/oder eines Ammonium-, Alkali- oder Erdalkalimetallsalzes einer organischen Mono-, Di- oder Tricarbonsäure verwendet, wobei die durch das Polyalkylenglykol funktionalisierten Proteine in Schritt d) in einem ansteigenden Konzentrationsgradienten des Elutionsmittels desorbiert werden, wobei die Salzkonzentration abnimmt.

Unter Desorption der durch das Polyalkylenglykol funktionalisierten Proteine in einem ansteigenden Konzentrationsgradienten wird nachfolgend verstanden, daß die Konzentration des Salzes bzw. der Salze in der wäßrigen Lösung des Elutionsmittels als Funktion der Dauer der Elution einer bestimmten Konzentration-Zeit-Funktion folgend erniedrigt wird. Besonders bevorzugt ist erfindungsgemäß eine lineare Erniedrigung der Salzkonzentration oder eine Erniedrigung der Salzkonzentration in Form eines Stufenprofils.

Als anorganische Ammonium-, Alkali- bzw. Erdalkalimetallsalze haben sich besonders Salze aus der Gruppe der Halogenide, Sulfate oder Phosphate bewährt, während unter den Ammonium-, Alkali- bzw. Erdalkalimetallsalzen der vorgenannten Mono-, Di- oder Tricarbonsäuren Salze der Ameisen-, Essig-, Capron-, Glycol-, Milch-, Äpfel-, Wein-, Fumar-, Malein-, Bernstein-, Oxal-, Malon-, Ascorbin-, Glucuron-, alpha-Ketoglutar-, (Iso)Citronen-, Triallyl- oder Aconitsäure bevorzugt sind.

Besonders bevorzugt wird für das erfindungsgemäße Verfahren ein Protein ausgewählt aus der Gruppe, umfassend Immunoglobuline, Insuline, Interferone, Albumine, wie Lactalbumin, Ovalbumin, bovines Serumalbumin, Myelopoietin, Erythropoietin, Trichosanthin, Tumor-Nekrose-Faktoren, oder Enzyme, wie Methioninase, Ribonukleasen, Staphylokinasen öder Lysozym, oder monoklonale bzw. rekombinante Antikörper, verwendet.

Bei einer besonders bevorzugten Ausführungsform des Verfahrens ist das Protein Lysozym und das Reaktionsprodukt des Proteins ein Gemisch bestehend aus einfach, zweifach und dreifach durch Polyethylenglykol funktionalisiertem Lysozym. Bei der vorgenannten Ausführungsform ist es möglich, alle vier Komponenten durch das erfindungsgemäße Verfahren aufzutrennen, zu isolieren und deren quantitativen Anteil an dem Gemisch der Pegylierungsprodukte zu bestimmen.

Die vorliegende Erfindung wird anhand der Figuren und anhand des folgenden, nicht einschränkenden Beispiels näher erläutert:

### Dabei zeigen

- Figur 1:: ein Chromatogramm eines Reaktionsansatzes aus der Umsetzung von Lysozym mit 5-kDa-Polyethylenglykol bei einer Gradientenelution, wobei das Volumen der 0,20-µm-Celluloseacetatmembran 1 ml beträgt,
- Figur 2:: ein Chromatogramm eines Reaktionsansatzes aus der Umsetzung von Lysozym mit 10-kDa-Polyethylenglykol bei einer Gradientenelution, wobei das Volumen der 0,20-µm-Celluloseacetatmembran 1 ml beträgt,
- Figur 3:: ein Chromatogramm eines Reaktionsansatzes aus der Umsetzung von Lysozym mit 30-kDa-Polyethylenglykol bei einem Volumen der 0,20-µm-Celluloseacetatmembran von 1 ml und bei einer Gradientenelution,
- Figur 4:: ein Chromatogramm des Reaktionsansatzes aus der Umsetzung von Lysozym mit 5-kDa-Polyethylenglykol bei einer Gradientenelution, wobei das Volumen der 0,20-µm-Celluloseacetatmembran 0,5 ml beträgt, und
- Figur 5:: ein Chromatogramm des Reaktionsansatzes nach Figur 4 bei einer Stufenelution, wobei das Volumen der 0,20-µm-Celluloseacetatmembran 0,5 ml beträgt.

### Beispiel:

### Pegylierung von Lysozym

Als Edukte für die Pegylierung wurden das Polyethylenglykol SUNBRIGHT^{®} ME-050AL mit aktivierten Aldehydgruppen und einem mittleren Molekulargewicht von 5.000 Da (Dalton) der Firma NOF Europe, Niederlande, Chargen-Nr. M83596, und Lysozym mit einem mittleren Molekulargewicht von 14.700 Da der Firma SIGMA Aldrich, Deutschland, Best.-Nr. L6876-100G, Chargen-Nr: 088K1358, sowie Natriumcyanoborhydrid NaCN(BH₃) der Firma FLUKA Buchs, Schweiz, Best.-Nr. 71435, Chargen-Nr. 1259328, verwendet. Lysozym und das Polyethylenglykol (PEG) wurden jeweils in einem 10-mmol/l-Citratpuffer (pH 8,0) gelöst. Der lysozymhaltigen Lösung wurde Natriumcyanoborhydrid zugegeben. Die Reaktion startete bei Raumtemperatur unmittelbar nach Mischung der lysozym- und NaCN(BH₃)-haltigen Lösung mit der PEG-haltigen Lösung. Die eingesetzten Mengen wurden so gewählt, daß im Reaktionsansatz die Lysozym-Konzentration 2 mg/ml betrug und daß das Stoffmengenverhältnis von PEG zu Lysozym 3:1 betrug. Es wurden in einem Ansatz 200 mg Lysozym, 140 mg PEG und 200 mg Natriumcyanoborhydrid eingesetzt. Die Komponenten wurden für 20 h bei Raumtemperatur umgesetzt und anschließend für 8 h bei 4 °C gelagert. Danach wurde das Gemisch der Pegylierungsprodukte und des nicht umgesetzten Lysozyms mit einer Serienschaltung von stark sauren Kationenaustauschern gereinigt.

Hierbei wurden vier in Serie geschaltete Membranadsorber des Sartobind^{®}-S-Typs (Stark saurer Kationenaustauscher mit Sulfonsäuregruppen auf einer Cellulosehydratmembran) der Firma Sartorius-Stedim Biotech GmbH, Best.-Nr. S 15X, verwendet. Die Adsorber wurden mittels einer geeigneten Schlauchquetschpumpe mit 100 ml eines 10 mmol/l-Citratpuffers (pH = 4,0, Puffer 1) gespült. 25 ml der jeweiligen Reaktionsansätze wurden auf die Adsorber aufgegeben. Die einzelnen Fraktionen an nicht umgesetztem Lysozym sowie an einfach und zwei- bzw. dreifach pegyliertem Lysozym wurden durch einen Stufengradienten von 0 mol/l NaCl in Puffer 1 bis 1 mol/l NaCl in Puffer 1 (entspricht Puffer 2) eluiert.

In der ersten Stufe bei 15 % Puffer 2 wurden das zwei- bzw. dreifach pegylierte Lysozym, in der zweiten Stufe bei 35 % Puffer 2 wurde das einfach pegylierte Lysozym und in der dritten Stufe bei 100 % Puffer 2 wurde das nicht umgesetzte Lysozym isoliert.

Die in diesen Stufen isolierten Fraktionen wurden durch Größenausschlußchromatographie an einer "G3000 SwXL"-Säule der Firma TOSOH Bioscience, Stuttgart, Deutschland, durch lineare Elution mit einem Laufmittel, bestehend aus 0,1 mol/l Natriumsulfat und 0,1 mol/l Natriumphosphat (pH = 7,0) nach ihrer Größe bzw. Molmasse aufgetrennt.

Desweiteren wurden die Fraktionen durch analytische Gelelektrophorese in einem 12-%- Polyacrylamidgel der Fa. ANAMED Deutschland (Best.-Nr. TG12112) und in einer Elektrophoresekammer "Elphor Vario 2" der Firma Bender und Hobein, Heidelberg, Deutschland, aufgetrennt. Eine anschließende Färbung mit Bariumiodid zum Nachweis von Polyethylenglykol und anschließende Färbung mit "Coomassie-Blue" zum Nachweis von pegylierten bzw. nicht pegylierten Proteinen bestätigte die durch Größenausschlußchromatographie erhaltenen Befunde.

Analoge Umsetzungen wurden auch für Polyethylenglykole mit einem mittleren Molekulargewicht von 10.000 Da bzw. 30.000 Da (SUNBRIGHT^{®} Best.-Nr. ME-100 AL (Charge M999653), SUNBRIGHT^{®} Best.-Nr. ME 300 AL (Charge M4659)) durchgeführt.

Nach diesen unabhängigen chromatographischen und gelelektrophoretischen Vergleichsanalysen wiesen die gereinigten Reaktionsansätze unter Einsatz von 5-kDa-, 10-kDa- bzw. 30-kDa-PEG jeweils ein Gemisch von nicht pegyliertem Lysozym sowie von einfach, zweifach und dreifach pegyliertem Lysozym auf. Die Ansätze werden nachfolgend als 5-kDa-, 10-kDa- bzw. 30kDa-PEG-Ansatz bezeichnet.

### Vergleich verschiedener mikroporöser Membranmaterialien mit einer mikroporösen Celluloseacetatmembran für die Auftrennung des 5-kDa-, 10-kDa- bzw. 30-kDa-PEG-Ansatzes in seine Einzelkomponenten

Als Equilibrierungs- und Bindungspuffer (nachfolgend "Puffer A") wurde ein Puffer aus 1 mol/l Trinatriumcitrat (bestehend aus 192,12 g Citronensäure der Fa. Merck, Darmstadt, eingestellt mit 1 mol/l NaOH) mit einem pH-Wert von 8,0 verwendet. Als Elutionspuffer (nachfolgend "Puffer B") wurde ein Puffer aus 0,1 mol/l Trinatriumcitrat (bestehend aus 19,21 g Citronensäure der Fa. Merck, Darmstadt, eingestellt mit 1 mol/l NaOH) mit einem pH-Wert von 8,0 verwendet. 0,5 ml des aufzutrennenden Gemisches aus Lysozym und seinen Pegylierungsprodukten (Einfach, zweifach und dreifach pegyliertes Lysozym) wurden mittels einer geeigneten Probenschleife aufgetragen und in einer FPLC-Anlage ÄKTAprime^{®} plus der Firma GE Healthcare, USA, wie unter Tabelle 2, Schritt 3, beschrieben, analysiert.

Es wurden die folgenden mikroporösen Membranen für die Auftrennung der vorgenannten Gemische verwendet, um ihre Trennleistung mit der für das erfindungsgemäße Verfahren verwendeten mikroporösen Celluloseacetatmembran zu vergleichen (vgl. Tabelle 1).

**Tabelle 1:**

| Membrantyp der Sartorius Stedim Biotech GmbH | Porengröße / µm | Typ-Nr. | Chargen-Nr. |
|---|---|---|---|
| 1) Celluloseacetat (CA) | 0,45 | 11106 | -100------G |
| 2) Celluloseacetat (CA) | 0,20 | 11107 | -100------G |
| 3) Polyamid (PA) | 0,20 | 25007 | -142------N |
| 4) Polyethersulfon (PESU) | 0,20 | 15407 | --50----MIN |
| 5) Cellulosenitrat (CN) | 0,45 | 11306 | 142------G |
| 6) regenerierte Cellulose (RC) | 0,20 | 18407 | 142------G |
| 7) Sartobind^{®} S | 3,00 | 94IEXS42-001 | ** |
| 8) Sartobind^{®} Phenyl | 3,00 | * | ** |

| | | | |
|---|---|---|---|
| * Die hier verwendete Membran wurde aus einer kommerziell erhältlichen Capsule der Firma Sartorius Stedim Biotech GmbH (Best.-Nr. 96HICP42E9BFF) entnommen und wie beschrieben verwendet. ** keine Chargenangabe vorhanden. Es handelt sich um Cellulosehydratmembranen mit Sulfonsäureliganden (S-Typ) bzw. mit Phenylliganden abgeleitet von Anilin (Phenyl-Typ). | | | |

Aus den in der Tabelle 1 aufgeführten Membranen wurden je drei Ronden mit einem Durchmesser von 30 mm gestanzt und in geeignete Halter eingebaut. Nach der vollständigen Benetzung der Membranen mit Puffer A wurden sie in eine FPLC-Chromatographie-Anlage des Typs ÄKTAprime^{®} integriert. Es folgte ein manueller Waschritt mit Puffer A.

Für den Vergleich der Membranen wurde ein automatisiertes Programm erstellt (vgl. Tabelle 2). Eluiert wurde über die angegebenen Volumina in ml in einem absteigenden Gradienten von 100 % bis 0 % Puffer A. Nach dem Elutionsschritt folgte ein weiterer Waschschritt mit Puffer B.

**Tabelle 2:**

| Schritt | Volumen / ml | Konzentration an Puffer B (%) | Probenaufgabe |
|---|---|---|---|
| 1 | 0,0 | 0 | - |
| 2 | 5,0 | 0 | - |
| 3 | 5,1 | 0 | Probenaufgabe |
| 4 | 10,0 | 0 | - |
| 5 | 100,0 | 90 | - |
| 6 | 100,1 | 100 | - |
| 7 | 110,0 | 100 | - |
| 8 | 110,1 | 0 | - |
| 9 | 120 | 0 | - |

Das Programm führte in Schritt 1 bis 3 einen Waschschritt der eingebauten Membran durch, in Schritt 3 erfolgte gleichzeitig die Aufgabe der zu analysierenden Probe aus Lysozym und seinen Pegylierungsprodukten durch Verwendung eines entsprechend geschalteten Ventils, so daß das zu analysierende Gemisch auf die Membran aufgetragen wurde. Zwischen Schritt 4 und 5 wurde durch ein entsprechend gesteuertes Proportionalventil ein linearer Gradient zwischen den Puffern A und B erzeugt. Da Puffer B eine geringere Ionenkonzentration als Puffer A aufweist, wird ein - bezogen auf den Salzgehalt - absteigender Gradient erzeugt.

Die Peaks der Chromatogramme wurden fraktioniert gesammelt und durch die oben beschriebenen Analysenverfahren des Größenausschlusses, des Kationenaustausches bzw. der Gelelektrophorese, analysiert und den verschiedenen Fraktionen, die Lysozym und seine Pegylierungsprodukte enthielten, zugeordnet.

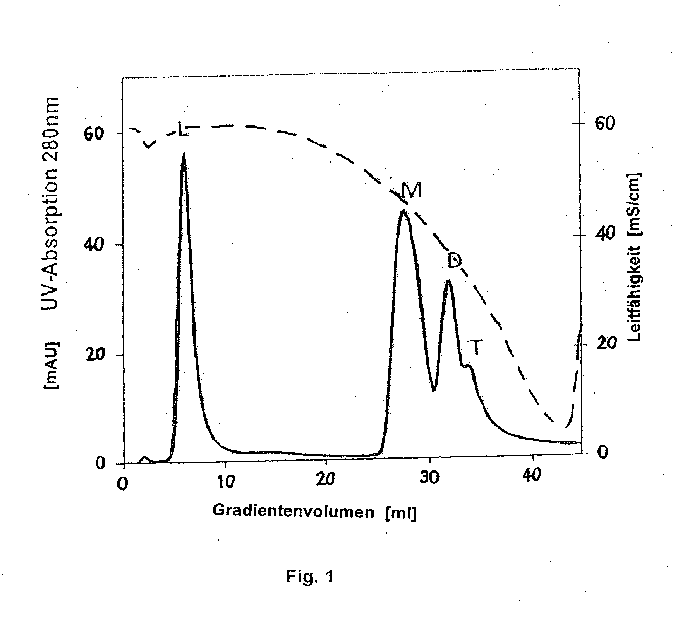

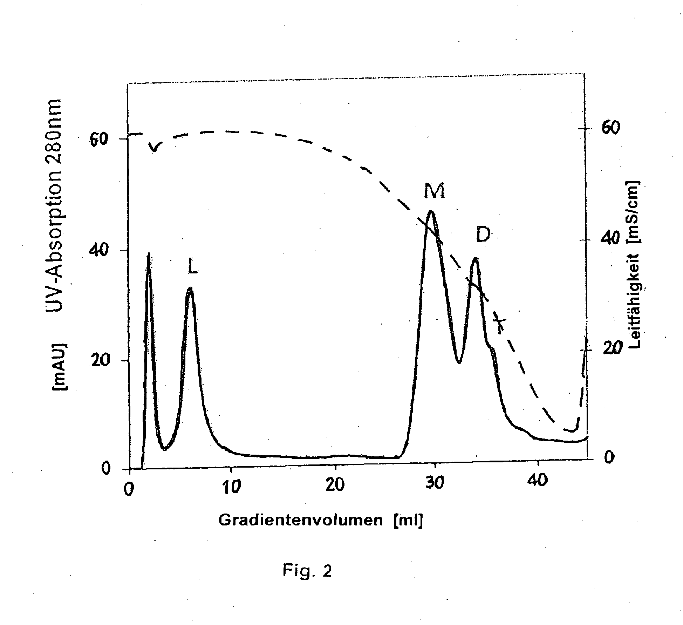

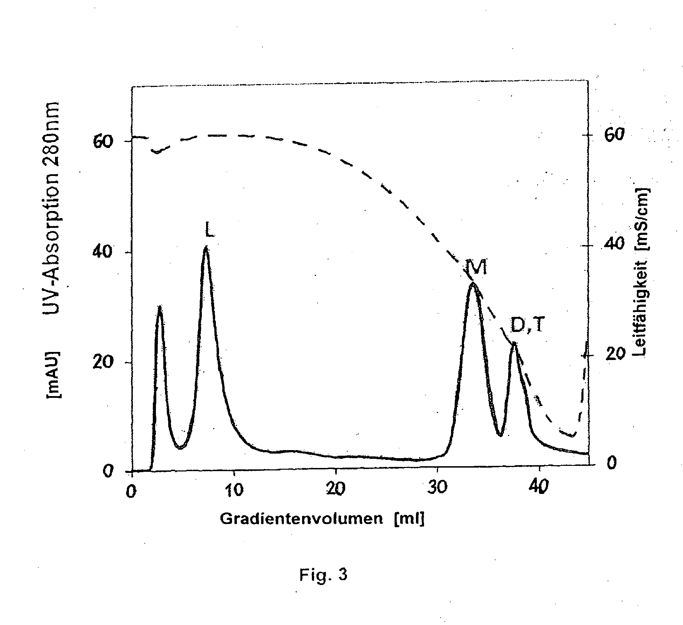

Die Figuren 1 bis 5 zeigen die Chromatogramme bei Durchführung des erfindungsgemäßen Verfahrens unter Verwendung der in Tabelle 1 unter Nr. 2) aufgeführten 0,20-µm-Celluloseacetatmembran. In den Figuren bezeichnet L Lysozym, M einfach pegyliertes Lysozym, D zweifach pegyliertes Lysozym und T dreifach pegyliertes Lysozym.

Gemäß den Figuren 1 bis 3 wurde der Verlauf der Auftrennung der Gemische anhand der Messung der UV-Absorption bei 280 nm im Ablauf des Adsorbers und der Leitfähigkeit des Eluats als Funktion der Dauer des automatisierten Programms nach Tabelle 2, d. h. als Funktion des Gradientenvolumens, verfolgt, wobei das Volumen der Membran 1 ml betrug. Die Elution erfolgte in einem Gradienten unter Anwendung des Programmes nach Tabelle 2 bei einer Flußrate von 1 ml/min und einer Gradientenlänge von 30 ml. Der Verlauf der Leitfähigkeit spiegelt die zeitliche Abnahme der Salzkonzentration im Gradienten wider, welche durch die gleichzeitige Zunahme des Anteils an Puffer B mit geringerer Salzkonzentration als Puffer A bedingt ist.

Aus den Figuren 1 bis 3 geht hervor, daß nicht pegyliertes Lysozym L und einfach pegyliertes Lysozym M zuverlässig von dem Gemisch aus zweifach (D) und dreifach pegyliertem Lysozym T abgetrennt werden können.

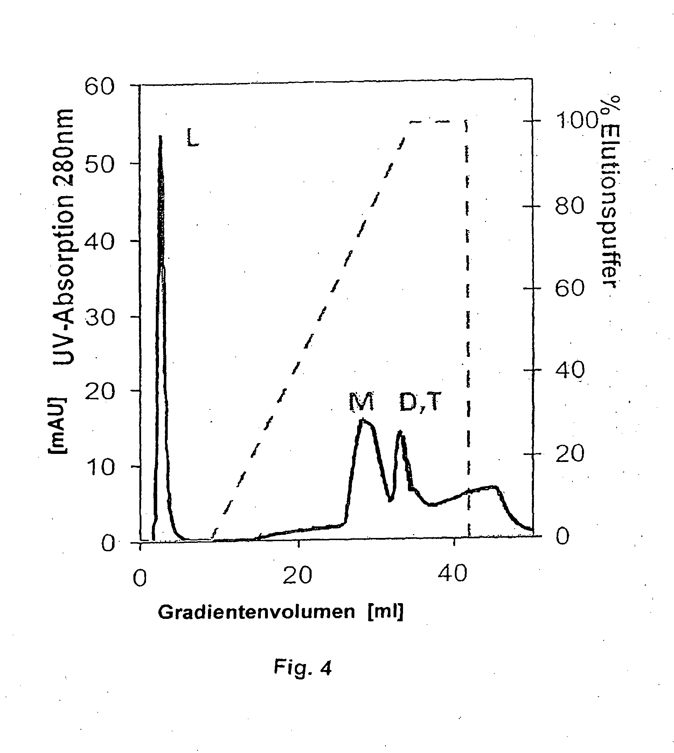

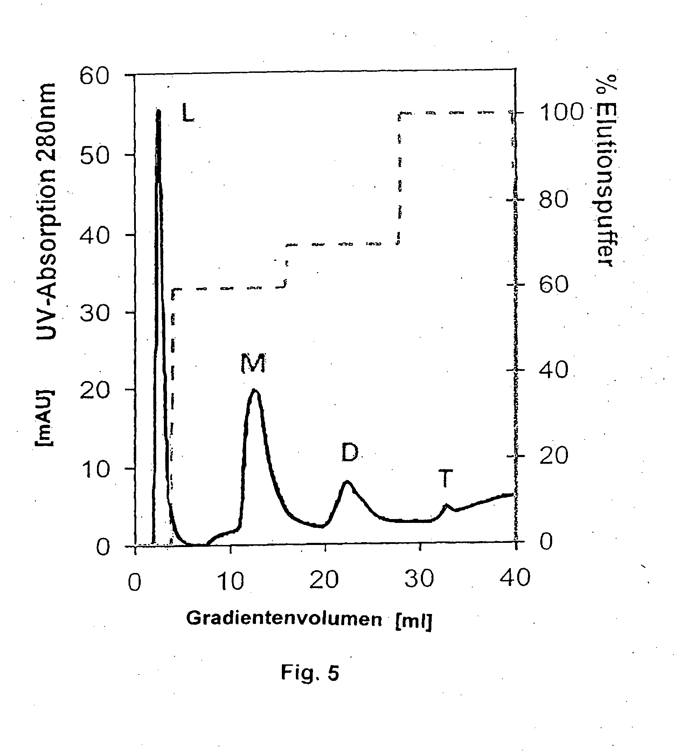

Gemäß den Figuren 4 und 5 wurde der Verlauf der Auftrennung des 5-kDa-PEG-Ansatzes in seine Einzelkomponenten als Funktion des Gradientenvolumens in ml anhand der UV-Absorption bei 280 nm und als Funktion von "% Elutionspuffer", d. h. des Anteiles an Puffer B in der Elutionslösung, verfolgt. Hierbei betrug das Membranvolumen 0,5 ml, wobei Figur 4 eine lineare Gradientenelution zeigt, während Figur 5 eine optimierte Stufenelution mit drei Plateaus zeigt.

Wird die Stofftrennung in dem in Figur 4 gezeigten linear ansteigendem Gradienten an Puffer B durchgeführt, ist es möglich, nicht pegyliertes Lysozym und einfach pegyliertes Lysozym zuverlässig von dem Gemisch aus zweifach und dreifach pegyliertem Lysozym abzutrennen. Nach Figur 5 ist für den 5-kDa-Reaktionsansatz im Gegensatz zu dem Ansatz nach Figur 1 neben der Abtrennung von nicht pegyliertem und einfach pegyliertem Lysozym auch die Auftrennung des Gemisches aus zweifach und dreifach pegyliertem Lysozym möglich, wenn der in Figur 5 gezeigte optimierte Stufengradient eingestellt wird. Als Puffer wurde hierbei eine 1,7-molarer Kaliumphosphatpuffer mit einem pH-Wert von 8,6 verwendet. Die Flußrate betrug 10 ml/min und die Gradientenlänge 30 ml. Die drei Stufen des Gradienten betrugen jeweils 60 %, 70 % und 100 % (jeweils 13 ml) des Puffers B.

Der Stufenelutionsgradient nach Figur 5 erlaubt die vollständige Auftrennung eines Gemisches aus Lysozym und seinen ein-, zwei- und dreifach pegylierten Reaktionsprodukten in die Einzelkomponenten.

Außerdem ist es anhand des Chromatogramms nach Figur 5 aufgrund der erreichten Basislinientrennungen möglich, durch Integration der einzelnen Peak-Flächen und durch Vergleich mit den entsprechenden Referenzsubstanzen die in dem Reaktionsansatz enthaltenen individuellen Mengen an nicht umgesetztem Lysozym und seinen einfach, zweifach und dreifach pegylierten Derivaten quantitativ zu bestimmen.

Vergleichsversuche wurden auch für die anderen Membranmaterialien 3) bis 8) der Tabelle 1 unter analogen Bedingungen durchgeführt. Die Ergebnisse zeigt Tabelle 3.

**Tab. 3 :**

| Membrantyp (vgl. Tab. 1) | Bindungsvermögen für Lysozym | Bindungsvermögen für Pegylierungsprodukte |
|---|---|---|
| Celluloseacetat (CA) | 0 | + / + |
| 2) Celluloseacetat (CA) | 0 | + / + |
| 3) Polyamid (PA) | + / - | + / + |
| 4) Polyethersulfon (PESU) | 0 | 0 |
| 5) Cellulosenitrat (CN) | + / - | + / - |
| 6) regenerierte Cellulose (RC) | 0 | 0 |
| 7) Sartobind^{®} S | 0 | 0 |
| 8) Sartobind^{®} Phenyl | + / - | + / + |

Das Symbol "0" bedeutet, daß die betreffende Komponente nicht an das untersuchte Membranmaterial adsorbiert wird. Ein Pluszeichen vor dem Schrägstrich bedeutet eine Bindung an das Membranmaterial, ein Pluszeichen hinter dem Schrägstrich bedeutet, daß die Komponenten von dem Membranmaterial eluiert werden können. Ein Minuszeichen hinter dem Schrägstrich bedeutet, daß die betreffende Komponente nicht von dem Membranmaterial eluiert werden kann. Aus Tabelle 3 ist ersichtlich, daß ausschließlich bei den beiden Celluloseacetatmembranen 1) und 2) keine Bindung des nicht pegylierten Lysozyms auftritt, aber die Pegylierungsprodukte des Lysozyms von den Membranen eluiert werden können, wenn die Ionenkonzentration abgesenkt wird. Die Celluloseacetatmembranen 1) und 2) haben eine Durchbruchskapazität für einfach pegyliertes Lysozym (bezogen auf das 5-kDa-Polyethylenglykol, mit welchem Lysozym umgesetzt wurde) von 0,042 mg/cm², entsprechend 1,7 mg an einfach pegyliertem Lysozym pro ml Membranvolumen. Bei allen anderen untersuchten Membranen 4), 6) und 7) erfolgte entweder keine Bindung der untersuchten Komponenten oder das nicht pegylierte Lysozym wurde zwar adsorbiert, konnte nachfolgend aber nicht desorbiert werden (vgl. Membranen 3), 5) und 8)).

Ein weiterer Vorteil des erfindungsgemäßen Verfahrens besteht in der Kosteneinsparung bei Wiederverwendung des preiswerten Celluloseacetatmembranmaterials für nachfolgende Trennoperationen.

## Patentansprüche

1. Verfahren zur Auftrennung eines Gemisches aus einem Protein und seinem Reaktionsprodukt mit einem Polyalkylenglykol, umfassend die Schritte:
a) Bereitstellen eines Gemisches umfassend das Protein und das Reaktionsprodukt in einem Fluid,
b) Kontaktieren des Gemisches aus Schritt a) mit einer mikroporösen Celluloseacetatmembran unter Adsorbieren des Reaktionsprodukts an der Celluloseacetatmembran, wobei das Protein nicht an der Celluloseacetatmembran adsorbiert wird,
c) Abtrennen des Proteins von der Celluloseacetatmembran und
d) Desorbieren des Reaktionsprodukts von der Celluloseacetatmembran,
wobei das Reaktionsprodukt aus der Gruppe, die das einfach oder mehrfach durch das Polyalkylenglykol funktionalisierte Protein oder Gemische davon umfaßt, ausgewählt ist.

2. Verfahren nach Anspruch 1, wobei das Reaktionsprodukt ein Gemisch ist, welches das einfach und mehrfach durch das Polyalkylenglykol funktionalisierte Protein umfaßt, und in Schritt d) die durch das Polyalkylenglykol funktionalisierten Proteine nacheinander einzeln mit einem Elutionsmittel von der Celluloseacetatmembran desorbiert werden.

3. Verfahren nach einem der Ansprüche 1 oder 2, wobei die Celluloseacetatmembran aus Cellulosemonoacetat, Cellulosediacetat, Cellulosetriacetat oder Gemischen davon besteht.

4. Verfahren nach Anspruch 3, wobei die Porengröße der Celluloseacetatmembran zwischen 0,1 und 15 µm beträgt.

5. Verfahren nach einem der vorhergehenden Ansprüche 2 bis 4, wobei als Elutionsmittel in Schritt d) eine wäßrige Lösung eines anorganischen Ammonium-, Alkali- bzw. Erdalkalimetallsalzes und/oder eines Ammonium-, Alkali- oder Erdalkalimetallsalzes einer organischen Mono-, Di- oder Tricarbonsäure verwendet wird und wobei die durch das Polyalkylenglykol funktionalisierten Proteine in Schritt d) in einem ansteigenden Konzentrationsgradienten des Elutionsmittels desorbiert werden.

6. Verfahren nach einem der vorhergehenden Ansprüche, wobei das Polyalkylenglykol ein Polyethylenglykol mit einem mittleren Molekulargewicht von 5 bis 30 kDa ist.

7. Verfahren nach einem der vorhergehenden Ansprüche, wobei das Protein ausgewählt ist aus der Gruppe, umfassend Immunoglobuline, Insuline, Interferone, Albumine, wie Lactalbumin, Ovalbumin, bovines Serumalbumin, Myelopoietin, Erythropoietin, Trichosanthin, Tumor-Nekrose-Faktoren, oder Enzyme, wie Methioninase, Ribonukleasen, Staphylokinasen oder Lysozym, oder monoklonale bzw. rekombinante Antikörper.

8. Verfahren nach einem der vorhergehenden Ansprüche 6 bis 7, wobei das Protein Lysozym ist und das Reaktionsprodukt ein Gemisch bestehend aus einfach, zweifach und dreifach durch Polyethylenglykol funktionalisiertem Lysozym ist.

## Claims

1. Method of separating a mixture consisting of a protein and its reaction product with a polyalkylene glycol, comprising the steps of:
a) provdiing a mixture comprising the protein and the reaction product in a fluid,
b) contacting the mixture of step a) with a microporous cellulose acetate membrane under adsorption of the reaction product at the cellulose acetate membrane, wherein the protein is not adsorbed at the cellulose acetate membrane,
c) separating off the protein from the cellulose acetate membrane and
d) desorbing the reaction product from the cellulose acetate membrane, wherein the reaction product is selected from the group which comprises the protein, which is functionalised once or multiple times by the polyalkylene glycol, or mixtures thereof.

2. Method according to claim 1, wherein the reaction product is a mixture which comprises the protein functionalised once and multiple times by the polyalkylene glycol and in step d) the proteins functionalised by the polyalkylene glycol are desorbed from the cellulose acetate membrane individually in succession by an elution medium.

3. Method according to one of claims 1 and 2, wherein the cellulose acetate membrane consists of cellulose monoacetate, cellulose diacetate, cellulose triacetate or mixtures thereof.

4. Method according to claim 3, wherein the pore size of the cellulose acetate membrane is between 0.1 and 15 microns.

5. Method according to any one of the preceding claims 2 to 4, wherein an aqueous solution of an inorganic ammonium, alkali or earth-alkali metal salt and/or an ammonium, alkali or earth-alkali metal salt of an organic monocarboxylic acid, dicarboxylic acid or tricarboxylic acid is used as elution medium in step d) and wherein the proteins functionalised by the polyalkylene glycol are desorbed in step d) in an increasing concentration gradient of the elution medium.

6. Method according to any one of the preceding claims, wherein the polyalkylene glycol is a polyethylene glycol with a mean molecular weight of 5 to 30 kDa.

7. Method according to any one of the preceding claims, wherein the protein is selected from the group comprising immunoglobulins, insulins, interferons, albumins, such as lactalbumin, ovalbumin, bovine serum albumin, myelopoietin, erythropoietin, trichosanthin, tumor-necrose factors or enzymes, such as methioninase, ribonucleases, staphylokinases or lysozyme, or monoclonal or recombinant antibodies.

8. Method according to any one of the preceding claims 6 and 7, wherein the protein is lysozyme and the reaction product is a mixture consisting of lysozyme functionalised once, twice and three times by polyethylene glycol.

## Revendications

1. Procédé pour séparer un mélange constitué d'une protéine et de son produit de réaction à l'aide d'un polyalkylène glycol, comprenant les étapes :
a) de mise à disposition d'un mélange comprenant la protéine et son produit de réaction dans un fluide,
b) de mise en contact du mélange provenant de l'étape a) avec une membrane en acétate de cellulose microporeuse en adsorbant le produit de réaction sur la membrane en acétate de cellulose, la protéine n'étant pas adsorbée sur la membrane en acétate de cellulose,
c) de séparation de la protéine de la membrane en acétate de cellulose, et
d) de désorption du produit de réaction de la membrane en acétate de cellulose,
le produit de réaction étant choisi dans le groupe qui comprend la protéine une ou plusieurs fois fonctionnalisée par le polyalkylène glycol ou des mélanges de celles-ci.

2. Procédé selon la revendication 1, dans lequel le produit de réaction est un mélange, lequel comprend la protéine une ou plusieurs fois fonctionnalisée par le polyalkylène glycol, et dans lequel, dans l'étape d), les protéines fonctionnalisées par le polyalkylène glycol sont individuellement désorbées les unes après les autres de la membrane en acétate de cellulose avec un produit d'élution.

3. Procédé selon l'une des revendications 1 ou 2, dans lequel la membrane en acétate de cellulose est constituée de monoacétate de cellulose, de diacétate de cellulose, de triacétate de cellulose ou de mélanges de ceux-ci.

4. Procédé selon la revendication 3, dans lequel la taille des pores de la membrane en acétate de cellulose est située entre 0,1 et 15 µm.

5. Procédé selon l'une des revendications précédentes 2 à 4, dans lequel, dans l'étape d), une solution aqueuse d'un sel inorganique d'ammonium, alcalin, respectivement, alcalino-terreux, et/ou d'un sel d'ammonium, alcalin ou alcalino-terreux d'un acide organique mono-, di- ou tricarboxylique est utilisée en tant que produit d'élution et dans lequel les protéines fonctionnalisées par le polyalkylène glycol sont désorbées dans l'étape d) par un gradient de concentration croissant du produit d'élution.

6. Procédé selon l'une des revendications précédentes, dans lequel le polyalkylène glycol est un polyéthylène glycol avec un poids moléculaire moyen de 5 à 30 kDa.

7. Procédé selon l'une des revendications précédentes, dans lequel la protéine est choisie dans le groupe comprenant des immunoglobulines, des insulines, des interférons, des albumines, telles que la lactalbumine, l'ovalbumine, l'albumine sérique bovine, la myélopoïétine, l'érythropoïétine, la trichosanthine, des facteurs de nécrose tumorale, ou des enzymes, telles que la méthioninase, les ribonucléases, les staphylokinases ou le lysozyme, ou des anticorps monoclonaux, respectivement, recombinants.

8. Procédé selon l'une des revendications précédentes 6 à 7, dans lequel la protéine est le lysozyme et le produit de réaction est un mélange constitué de lysozyme une fois, deux fois et trois fois fonctionnalisé par du polyéthylène glycol.
